# EUROPEAN PATENT SPECIFICATION

(11) **EP 2 047 795 B1**
(45) Date of publication and mention of the grant of the patent: **03.07.2013**
(21) Application number: 07791062.8
(22) Date of filing: 20.07.2007
(51) Int. Cl.: A61B 5/022

(54) **ELECTRONIC BLOOD PRESSURE MONITOR HAVING CUFF IN WHOSE INNER, PRESSURE IS ADEQUATELY ADJUSTED AND ITS CONTROL METHOD**
ELEKTRONISCHER BLUTDRUCKMONITOR MIT MANSCHETTE, MIT ENTSPRECHEND GEREGELTEM INNEREN DRUCK SOWIE STEUERVERFAHREN
DISPOSITIF ELECTRONIQUE DE MESURE DE LA PRESSION SANGUINE MUNI D'UN BRASSARD DONT LA PRESSION INTERNE EST AJUSTEE AVEC PRECISION, ET PROCEDE DE REGULATION ASSOCIE

(30) Priority: 03.08.2006 JP 2006212090
(43) Date of publication of application: 15.04.2009
(73) Proprietor: Omron Healthcare Co., Ltd., Ukyo-ku Kyoto-shi Kyoto 615-0084 (JP)
(72) Inventor: YAMAKOSHI, Kenichi, Kanazawa-shi Ishikawa 920-0953 (JP); TANAKA, Shinobu, Kanazawa-shi Ishikawa 920-1303 (JP); NOGAWA, Masamichi, Kanazawa-shi Ishikawa 920-0947 (JP); YAMAKOSHI, Takehiro, Kanazawa-shi Ishikawa 920-0953 (JP); SAWANOI, Yukiya, Kyoto-shi Kyoto 615-0084 (JP)
(74) Representative: Kilian Kilian & Partner
(86) International application number: PCT/JP2007/064324
(87) International publication number: WO 2008/015921

(56) References cited:
- EP-A1- 0 284 095
- JP-A- 59 156 325
- JP-A- 2003 135 412
- US-A- 4 524 777
- YAMAKOSHI K ET AL: "New oscillometric method for indirect measurement of systolic and mean arterial pressure in human finger. part 2" MEDICAL AND BIOLOGICAL ENGINEERING AND COMPUTING, SPRINGER, HEILDELBERG, DE, vol. 20, 1 January 1982 (1982-01-01), pages 314-318, XP009140190 ISSN: 0140-0118
- YAMAKOSHI K ET AL: "New oscillometric method for indirect measurement of systolic and mean arterial pressure in human finger. part 1" MEDICAL AND BIOLOGICAL ENGINEERING AND COMPUTING, SPRINGER, HEILDELBERG, DE, vol. 20, 1 January 1982 (1982-01-01), pages 307-313, XP009140189 ISSN: 0140-0118
- HYNDMAN B W ET AL: "The Navicon: a noninvasive monitor of instantaneous blood pressure using a compressible air-space servocontroller" PROCEEDINGS OF THE COMPUTERS IN CARDIOLOGY CONFERENCE. LONDON, SEPT. 5 19930905; 19930905 - 19930908 LOS ALAMITOS, IEEE COMP. SOC. PRESS, US LNKD- DOI:10.1109/CIC.1993.378481, vol. -, 5 September 1993 (1993-09-05), pages 153-156, XP010128861 ISBN: 978-0-8186-5470-1
- NISHIO T. ET AL.: 'Yoseki Hoshogata Renzoku Ketsuatsu Sokutei no Tame no Servo Mokuhyochi (VO) Ketteiho no Aratana Kokoromi (A new method for determining the servo reference value (Vo) by the volume-compensation method for non-invasive beat-by-beat blood pressure monitoring)' NIPPON SEITAII KOGAKU ZASSHI (DAI 45 KAI JAPANESE SOCIETY FOR MEDICAL AND BIOLOGICAL ENGINEERING TAIKAI RONBUNSHU) vol. 44, 15 May 2006, page 446, XP003020556

## Description

### TECHNICAL FIELD

The present invention relates to an electronic manometer and a method for controlling the same, in particular, to an electronic manometer for measuring blood pressure using a volume compensation method, and a method for controlling the same.

### BACKGROUND ART

Blood pressure is one index for analyzing cardiovascular disorder, where performing risk analysis based on the blood pressure is effective in preventing cardiovascular diseases such as stroke, cardiac arrest, and cardiac infarction. In particular, morning hypertension in which the blood pressure rises in early morning is related to heart disease, stroke, and the like. Furthermore, it is known that in the morning hypertension, a symptom in which the blood pressure rapidly rises from one hour to about one and a half hour after wakeup called morning surge has a causal association with stroke. Understanding the mutual relationship between time (lifestyle) and change in blood pressure is useful in risk analysis of the cardiovascular disease. Therefore, continuous measurement of the blood pressure over a long period of time is necessary.

In monitoring patients during surgery and after surgery, checking medicinal effects in treatment with antihypertensive drug, and the like, it is very important to continuously measure the blood pressure for every heart beat and to monitor the change in blood pressure.

The pulse wave waveform for every heart beat includes information having an extremely wide usage range in a medical field such as progress of arterial sclerosis and diagnosis of heart function, and it is also important to continuously record a fluctuation in a pulse wave waveform.

In a process of wrapping a cuff at a measurement site, pressurizing the pressure within the cuff to higher than a systolic blood pressure, and then gradually depressurizing the pressure within the cuff, the conventional manometer detects the pulsation generated in the artery with a pressure sensor via the cuff, and applies a predetermined algorithm to the pressure within the cuff and the magnitude of the pulsation at the time (pulse wave amplitude) to determine the systolic blood pressure and the diastolic blood pressure (oscillometric method). Such manometer utilizes a characteristic that the pulse wave amplitude rapidly increases when the internal pressure of the cuff is near the systolic blood pressure and rapidly decreases when near the diastolic blood pressure.

In the conventional manometer, a blood flow sound (Korotkov's sound) that appears or disappears depending on the change in blood flow during pressurization and depressurization of the internal pressure of the cuff is detected with a Korotkov's sound detector such as a microphone to determine the systolic blood pressure and the diastolic blood pressure (Korotkov's sound method). Such manometer utilizes the characteristic that the Korotkov's sound appears when the internal pressure of the cuff is at the systolic blood pressure, and disappears (or muffles) near the diastolic blood pressure.

In such blood pressure measurement method, the internal pressure of the cuff needs to be gradually depressurized to determine the systolic blood pressure and the diastolic blood pressure, and thus the measurement time for one measurement requires about thirty seconds at the fastest, and it is impossible to measure the blood pressure for every heart beat.

A method of measuring the blood pressure for every heart beat includes a method of inserting a catheter into the artery, and directly measuring the intra-arterial pressure by a pressure sensor connected to the catheter (direct method). The direct method inserts the catheter into the artery, and thus needs to be conducted under a supervision of a doctor, and also provides psychological and/or physical pain such as pain and/or risk of infection to the measurement subject (patient) and can be performed only under a specified environment such as during a surgery or after a surgery.

Under such situation, a technique of noninvasively and continuously measuring the blood pressure for every heart beat has been developed.

For such a technique, for example, a blood pressure measurement method called a tonometric method is known. Here, when the superficial artery such as a radial artery is compressed flat from the body surface, the pressure on the plane can be detected by the pressure sensor to measure the blood pressure using the principle that the pressure on the plane matches the intra-arterial pressure. Actually, it is impossible to compress only the artery, and the tissues of the body surface are also simultaneously compressed, and thus the pressure detected by the pressure sensor does not necessarily match the intra-arterial pressure. Therefore, in measuring the blood pressure through the tonometric method, correction needs to be made by the blood pressure value detected through other methods (e.g., oscillometric method). Furthermore, the sensor needs to be correctly arranged on the artery to flatly compress the artery, and thus it is very troublesome to handle, and is used only in a limited state such as during a surgery or when treating in an intensive case after the surgery.

A blood pressure measurement by volume compensation method is developed as disclosed in Japanese Unexamined Patent Publication No. 54-50175 (Patent Document 1) as a method for easy and convenient measurement.

The volume compensation method is a method of compressing the artery by means of the cuff from outside the body, equilibrating the compressed pressure (cuff pressure) and the intra-arterial pressure, that is, blood pressure by maintaining the volume per unit length of the pulsating artery constant, and
detecting the cuff pressure when such state is maintained to obtain a continuous blood pressure value. Since the arterial wall needs to be maintained in a no-load state (i.e., natural state not applied with pressure), two points of detecting that the artery is in the no-load state (detection of servo target value) and maintaining the relevant state (servo control) become important. In particular, determination of the servo target value directly influences the blood pressure measurement accuracy, and thus its determination is very important.

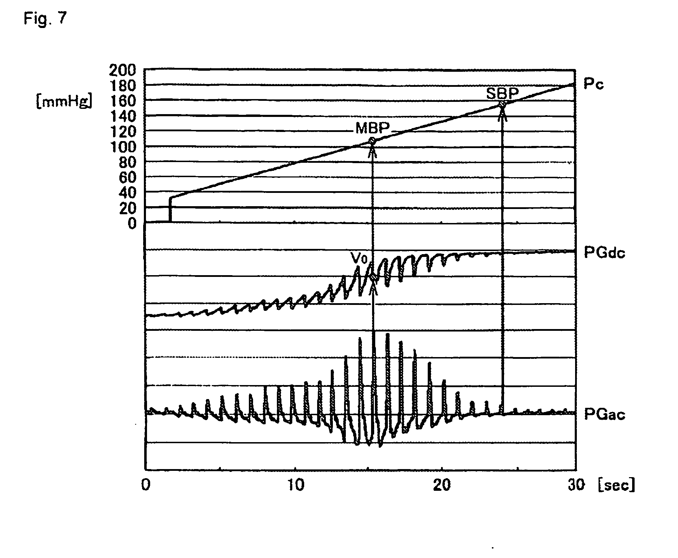

As examples of a technique related to the determination of the servo target value in the electronic manometer, Japanese Unexamined Patent Publication No. 59-156325 (Patent Document 2) and US 4,524,777 A disclose a technique of gradually compressing the artery with the cuff, and detecting the artery volume maximum point obtained at the relevant time to obtain the servo target value (control target value). The mode of determination of the control target value disclosed in the publication will be described with reference to Fig. 7. In Fig. 7, a cuff pressure is indicated as Pc, an output voltage (volume signal of the artery) of a photo-transistor is indicated as PGdc, and a pulse wave component of the volume signal is indicated as PGac.

In the technique disclosed in Patent Document 2 and US 4,524,777 A, while gradually pressurizing the cuff (3 to 4 mmHg/sec), the pulse wave component of the volume signal at the relevant time is acquired, and the maximum value thereof is detected. The internal pressure of the cuff at the point the maximum value is detected is set as the servo target value.
[Patent Document 1] Japanese Unexamined Patent Publication No. 54-50175
[Patent Document 2] Japanese Unexamined Patent Publication No. 59-156325.

EP 0 284 095 A1 discloses a device according to the preamble of claim 1.

### DISCLOSURE OF THE INVENTION

### PROBLEMS TO BE SOLVED BY THE INVENTION

In the technique disclosed In Patent Document 2 and US 4,524,777 A, an accurate servo target value (control target value) can be detected, but 30 seconds or more are required at minimum to detect the servo target value. Thus, 30 + α seconds or more are required until a first blood pressure determination from a start of measurement, and a measurement subject feels inconvenience.

During continuous blood pressure measurement, the servo target value sometimes fluctuates due to blood pressure fluctuation, stress, change in environment, and the like, and the servo target value needs to be redetected each time, but in this case as well, at least 30 seconds are required until the determination of the servo target value. Thus, the blood pressure measurement needs to be interrupted, and the control target value needs to be re-determined, whereby the blood pressure fluctuation during the continuous blood pressure measurement cannot be rapidly responded.

In view of the above situations, it is an object of the present invention to determine a control target value in a short period of time in an electronic manometer for measuring the blood pressure using a volume compensation method.

### MEANS FOR SOLVING THE PROBLEMS

An electronic manometer of the present invention as defined by claim 1 includes a cuff attached to a blood pressure measurement site of a measurement subject; a pressure adjustment unit for adjusting a pressure to apply on the cuff; a pressure detection unit for detecting a pressure within the cuff; an artery volume detection unit for detecting a volume of an artery of the measurement subject, which changes by a change in the pressure within the cuff and pulsation; and a measurement unit for controlling the volume of the artery of the measurement subject so as to be constant by causing the pressure adjustment unit to increase or decrease the pressure to apply on the cuff based on the change in the volume of the artery of the measurement subject, and measuring a blood pressure of the measurement subject from an increased or decreased value of the pressure to apply on the cuff; wherein the measurement unit includes a pressure maintaining unit for detecting the pressure within the cuff at which an amplitude of the change in the volume of the artery of the measurement subject becomes a maximum, and maintaining the pressure, a feedback control unit for operating the pressure adjustment unit based on the change in the volume of the artery of the measurement subject and feedback controlling so that the amplitude of the change in the volume of the artery of the measurement subject becomes a minimum, and an extracting unit for extracting the pressure within the cuff when the amplitude of the change in the volume of the artery of the measurement subject becomes a minimum by the control from the feedback control unit as the blood pressure of the measurement subject; and the pressure maintaining unit causes the artery volume detection unit to detect the volume of the artery of the measurement subject while superimposing a vibration of high frequency during a period of causing the pressure adjustment unit to raise the pressure to apply on the cuff or during a period to lower after raising to greater than or equal to a systolic blood pressure of the measurement subject to detect the pressure within the cuff at which the amplitude of the change in the volume of the artery of the measurement subject becomes a maximum.

In the electronic manometer of the present invention, the pressure maintaining unit detects the pressure within the cuff at which the amplitude of the change in the volume of the artery of the measurement subject becomes a maximum at a start of measurement of the blood pressure by the measurement unit and at a time other than at the start of measurement during the period of causing the pressure adjustment unit to lower the pressure to apply on the cuff after raising to greater than or equal to the systolic blood pressure of the measurement subject while the feedback controlling is performed by the feedback control unit.

Preferably, the time other than at the start of the measurement is when the blood pressure measured by the measurement unit exceeds a predetermined value or becomes lower than a specific value.

Preferably, the time other than at the start of the measurement is when an amount of change in the volume of the artery of the measurement subject detected by the artery volume detection unit exceeds a constant value during the measurement of the blood pressure by the measurement unit.

Preferably, the time other than at the start of the measurement is when the amount of change in the pressure within the cuff detected by the pressure detection unit exceeds a certain value during the measurement of the blood pressure by the measurement unit.

An operation unit for accepting an operation from outside is further preferably arranged; and the time other than at the start of the measurement is when the operation is made to the operation unit.

The pressure maintaining unit preferably detects the pressure within the cuff at which the amplitude of the change in the volume of the artery of the measurement subject becomes a maximum based on a detection result of the volume of the artery of the measurement subject by the artery volume detection unit in a period the pressure adjustment unit adjusts the pressure to apply on the cuff by one heart beat based on the control by the feedback control unit.

A method of controlling an electronic manometer of the present invention as defined by claim 7 is a method of controlling an electronic manometer including a cuff attached to a blood pressure measurement site of a measurement subject, the method causing the electronic manometer to execute the steps of detecting a pressure within the cuff at which an amplitude of change in a volume of an artery of the measurement subject becomes a maximum; maintaining the detected pressure within the cuff; adjusting a pressure to apply on the cuff based on the change in the volume of the artery of the measurement subject while performing a feedback control so that the amplitude of the change in the volume of the artery of the measurement subject becomes a minimum; and extracting the pressure within the cuff when the amplitude of the change in the volume of the artery of the measurement subject becomes a minimum by the feedback control as the blood pressure of the measurement subject; wherein the step of detecting the pressure within the cuff includes detecting the volume of the artery of the measurement subject while superimposing a vibration of high frequency during a period of raising the pressure to apply on the cuff or during a period of lowering after raising the pressure to apply on the cuff to greater than or equal to a systolic blood pressure of the measurement subject to detect the pressure within the cuff at which the amplitude of the change in the volume of the artery of the measurement subject becomes a maximum. In the method of controlling the electronic manometer of the present invention, the step of detecting the pressure within the cuff is executed at a start of blood pressure measurement in the electronic manometer and at a time other than at the start of the measurement during the period of lowering after raising the pressure to apply on the cuff to greater than or equal to the systolic blood pressure of the measurement subject while performing the feedback control.

According to the present invention, the pressure within the cuff when the amplitude of the change in the volume of the artery of the measurement subject detected with the vibration of high frequency superimposed on the pressure to apply on the cuff becomes a maximum is the pressure corresponding to the target value (control target value) to which the pressure within the cuff is to be maintained. According to the present invention, the pressure within the cuff at which the
amplitude of the change in the volume of the artery of the measurement subject becomes a maximum is detected at the start of measurement of blood pressure by the measurement unit and at a time other than at the start of measurement during the period of lowering after raising the pressure to apply on the cuff to greater than or equal to the systolic blood pressure of the measurement subject while performing the feedback control.

The pressure within the cuff when the change in the volume of the artery of the measurement subject becomes a maximum then can be obtained for every feedback control corresponding to one heart beat.

### BRIEF DESCRIPTION OF THE DRAWINGS

Fig. 1 is an overview diagram of an electronic manometer according to one embodiment of the present invention.
Fig. 2 is a view schematically showing a hardware configuration of the electronic manometer of Fig. 1.
Fig. 3 is a flowchart of a process executed in the electronic manometer shown in Fig. 1 when a power supply is turned ON to measure blood pressure.
Fig. 4 is a flowchart of a sub-routine for detecting a control target value in a process shown in Fig. 3.
Fig. 5 is a view showing data handled in a CPU of the electronic manometer shown in Fig. 1.
Fig. 6 is a flowchart of a variant of the process shown in Fig. 3.
Fig. 7 is a view for describing a mode of determining the control target value in a conventional electronic manometer.

### DESCRIPTION OF REFERENCE SYMBOLS

| | |
|---|---|
| 1 | Electronic manometer |
| 2 | Cuff |
| 3 | Tube |
| 4 | Display unit |
| 5 | Operation unit |
| 6 | Timer |
| 10 | CPU |
| 11, 12 | Memory |
| 13 | Oscillation circuit |
| 14 | Pump drive circuit |
| 15 | Valve drive circuit |
| 16 | Artery volume detection circuit |
| 20 | Air bag |
| 21 | Artery volume sensor |
| 30 | Power supply |
| 31 | Pressure sensor |
| 32 | Pump |
| 33 | Valve |
| 51 | Power switch |
| 52 | Measurement switch |
| 53 | Stop switch |
| 54 | Record call-out switch |

### BEST MODE FOR CARRYING OUT THE INVENTION

An embodiment of an electronic manometer according to the present invention will be described below with reference to the drawings.

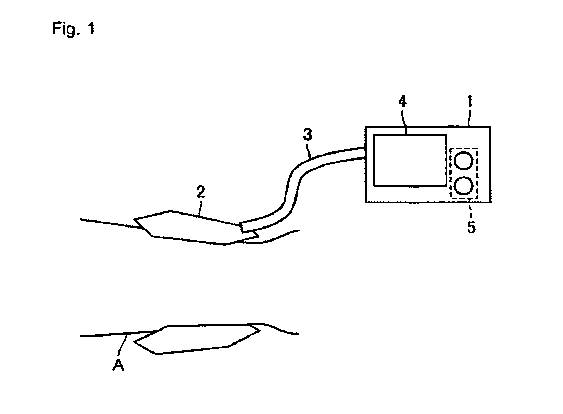

Fig. 1 is an overview diagram of an electronic manometer according to one embodiment of the present invention.

The electronic manometer 1 includes a cuff 2, and measures blood pressure of a measurement subject with the cuff 2 wrapped around a wrist A of the measurement subject. In the electronic manometer according to the present invention, a measurement site of the blood pressure is not limited to the wrist. The measurement sites may be a fingertip, an upper arm, and the like as long as a volume of an artery can be detected, as hereinafter described.

The electronic manometer 1 is arranged on a front surface with a display unit 4 and an operation unit 5 with a plurality of operation buttons. The electronic manometer 1 also includes a tube 3 for connecting a built-in pump (later-described pump 32) and the cuff 2. The cuff 2 internally includes an air bag.

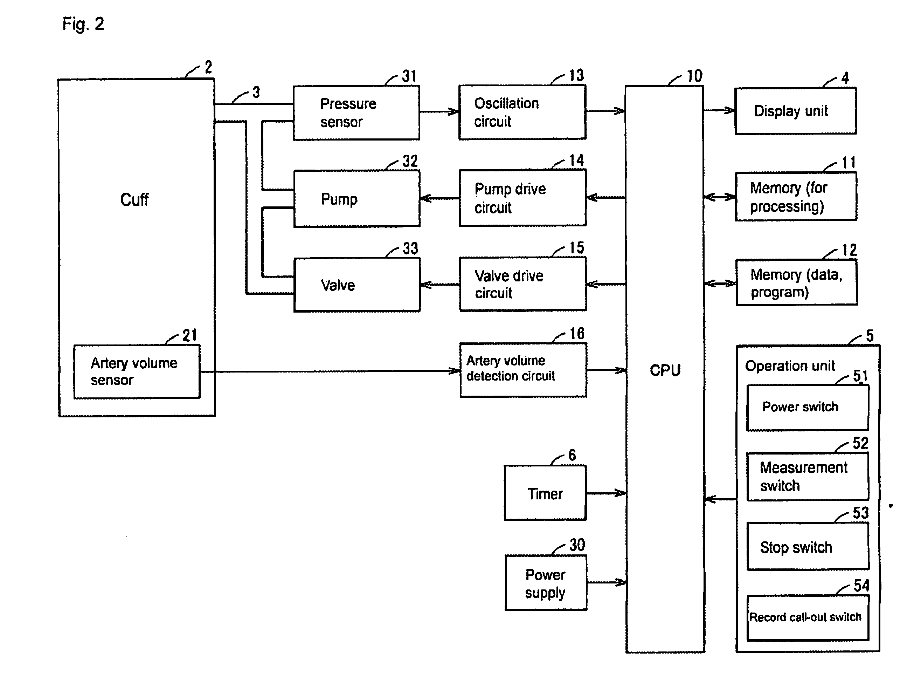

Fig. 2 is a view schematically showing a hardware configuration of the electronic manometer 1.
The electronic manometer 1 includes, in addition to the display unit 4 and the operation unit 5 discussed above, a CPU (Central Processing Unit) 10 for wholly controlling the operation of the electronic manometer 1, a memory 11 functioning as a work memory of the CPU 10, a memory 12 for storing various information (program and data), a timer 6, and a power supply 30 for supplying power to the CPU 10. The operation unit 5 includes a power switch 51 operated to switch ON/OFF of the power supply with respect to the electronic manometer 1, a measurement switch 52 operated to cause the electronic manometer 1 to start the measurement of the blood pressure, a stop switch 53 operated to cause the electronic manometer 1 to stop the measurement operation, and a record call-out switch 54 operated to call out the measurement result of the blood pressure and the like stored in the memory 12.

The electronic manometer 1 further includes, in addition to the cuff 2 and the tube 3 described above, a pressure sensor 31 for measuring the pressure of the air bag of the cuff 2, an oscillation circuit 13 for converting an output from the pressure sensor 31 to frequency and inputting the same to the CPU 10, a pump 32 for supplying air to the air bag of the cuff 2, a pump drive circuit 14 for driving the pump 32, a valve 33 for opening/closing a connecting portion of the tube 3 and the pump 32, and a valve drive circuit 15 for driving the valve 33. In the electronic manometer 1, the CPU 10 controls the operation of the pump drive circuit 14 and the valve drive circuit 15 to control the pressure of the air bag of the cuff 2.

The electronic manometer 1 further includes an artery volume sensor 21 attached to the cuff 2, and an artery volume detection circuit 16 input with a detection output of the artery volume sensor 21. The artery volume sensor 21 is a sensor for detecting the volume of the artery at the blood pressure measurement site of the measurement subject, and is configured by a photoelectric sensor and the like. The photoelectric sensor specifically includes a light emission diode, and a phototransistor arranged so as to face the light emission diode with the measurement site in between. The artery volume detection circuit 16 controls a light emission quantity of the light emission diode, and is input with a light receiving quantity of the phototransistor. Thus, in the artery volume detection circuit 16, the volume of the artery is detected based on a transmitted light quantity reaching the photodiode of the light emitted by the light emission diode, which is the light of absorption band of hemoglobin contained in the blood (red blood cell) flowing through the blood vessel. In the electronic manometer according to the present invention, the volume of the artery may be detected, other than by the photoelectric sensor, using existing methods such as an impedance plethysmograph.

In the electronic manometer 1, the blood pressure (systolic blood pressure, diastolic blood pressure) is detected using a volume compensation method. The blood pressure measurement through the volume compensation method obtains the blood pressure by applying external pressure to the artery from ex vivo, performing control to constantly equilibrate with the in vitro pressure (intra-arterial pressure, that is, blood pressure), maintaining the arterial wall in a no-load state, and measuring the in vitro pressure at that point. The blood pressure measurement using the volume compensation method is specifically described in Patent Document 1.

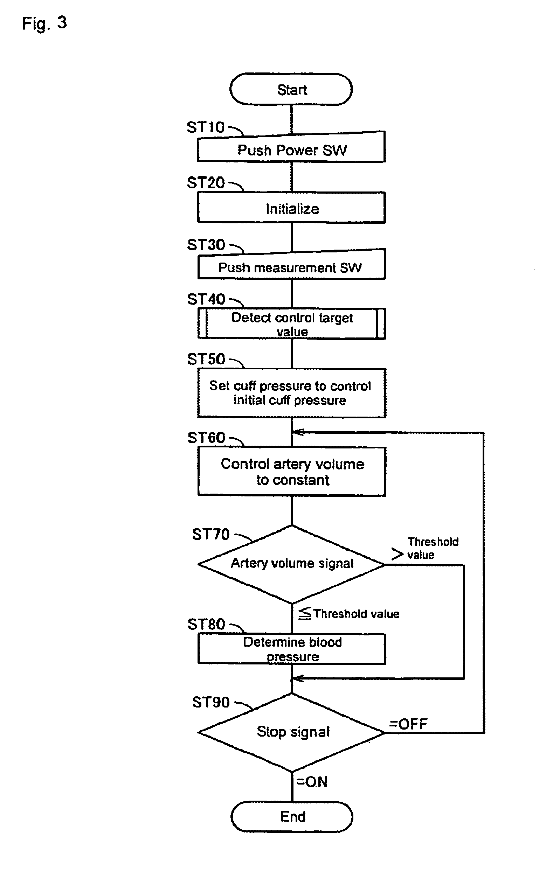

Fig. 3 is a flowchart of a process executed in the electronic manometer 1 when the power supply is turned ON to measure the blood pressure.

With reference to Fig. 3, first the power switch 51 is pushed by the measurement subject or a user in step ST10. Initialization is then performed in the electronic manometer 1 in step ST20. The initialization includes initialization of the memory 11. exhaust of air in the cuff 2, and correction of 0 mmHg of the pressure sensor 31 by the CPU 10.

When the measurement switch 52 is pushed (step ST30) after initialization, a control target value for the no-load state of the cuff 2 is detected in step ST40 in the electronic manometer 1. The processing content for detecting the control target value in step ST40 will be hereinafter described.

When the control target value is determined, the CPU 10 adjusts the pressure of the air bag of the cuff 2 to a control initial cuff pressure, to be hereinafter described, (step ST50), and then determines the blood pressure of the measurement subject while feedback controlling the pressure of the air bag of the cuff 2 so that the amplitude of an artery volume signal becomes a minimum (steps ST60 to ST80). The artery volume signal is a signal output from the artery volume detection circuit 16 to the CPU 10, and is a signal corresponding to the volume of the artery of the measurement subject.

In step ST60, the CPU 10 controls the pressure of the air bag of the cuff 2 so that the amplitude of the artery volume signal becomes a minimum based on the detection output of the artery volume detection circuit 16. When referring to "so that the amplitude of the artery volume signal becomes a minimum", it means so that the volume of the artery of the measurement subject becomes lower than or equal to a threshold value defined in advance. For instance, if the detection output of the artery volume detection circuit 16 infers that the volume of the artery of the measurement subject exceeds the threshold value, the CPU 2 controls the operation of the valve 33 so as to raise the pressure of the air bag of the cuff 2 according to the exceeding degree. In the present embodiment, when measuring the blood pressure, the pump 32 is constantly operated, and the pressure of the air bag of the cuff 2 is controlled by the opening/closing mode of the valve 33. The pressure of the air bag of the cuff 2 may be controlled by the driving mode of the pump 32 with the valve 33 constantly opened, or may be controlled by combining both the opening/closing mode of the valve 33 and the driving mode of the pump 32.

In step ST70, the CPU 10 determines whether or not the artery volume signal infers that the volume of the artery of the measurement subject is lower than or equal to the threshold value. If determining as lower than or equal to the threshold value, the CPU 10 decides the pressure of the air bag of the cuff 2 at the relevant point as the blood pressure of the measurement subject in step ST80, and proceeds the process to step ST90. If determining as exceeding the threshold value, the CPU 10 directly proceeds the process to step ST90 without executing the process of step ST80. In step ST80, the CPU 10 displays the determined blood pressure value on the display unit 4, or stores the same in the memory 12 (e.g., along with time at the relevant point).

In step ST90, the CPU 10 determines whether or not the stop signal is turned ON, and returns the process to step ST60 if determining as turned OFF and terminates the measurement if determining as turned ON. The stop signal is a signal for stopping the measurement of the blood pressure, and is turned OFF at the initialization of step ST20, and is turned ON when the stop switch 53 is pushed or after elapse of a predetermined time from the start of measurement of the blood pressure (pushing of measurement switch 52 in step ST30).

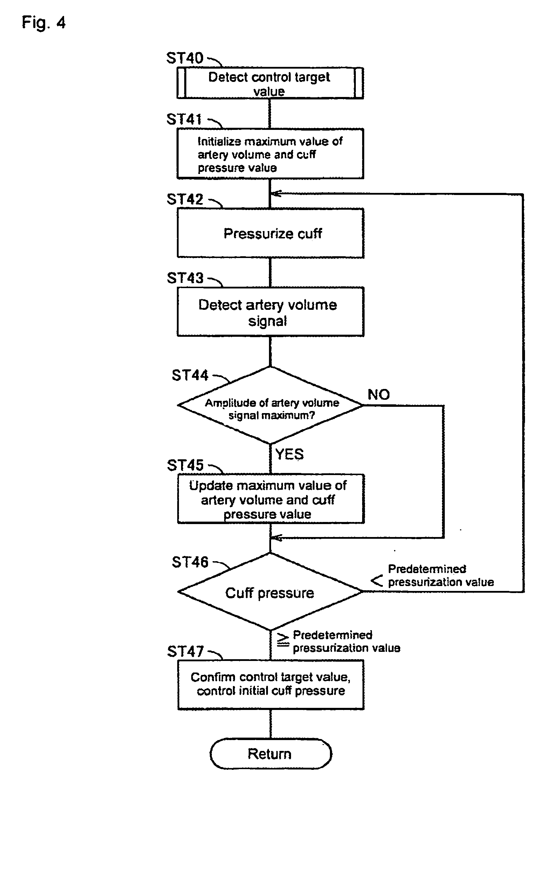

Fig. 4 is a flowchart of a sub-routine for detecting the control target value in step ST40.

With reference to Fig. 4, in the detection process of the control target value, the CPU 10 first initializes a maximum value of the artery volume (artery volume signal) stored in the memory 12 and a pressure value of the air bag of the cuff 2 at the point the maximum value is detected in step ST41.

In step ST42, the CPU 10 then pressurizes the air bag of the cuff 2. The pressurization of the air bag of the cuff 2 is performed not by simply raising the pressure linearly, but by superimposing a high frequency minimal pressure vibration (e.g., frequency of 20 Hz, amplitude of 10 mmHg) on a linear pressure raising waveform. The vibration of high frequency is superimposed on the pressure to apply on the cuff 2.

The CPU 10 detects the artery volume signal from the artery volume detection circuit 16 in step ST43, and determines whether or not the amplitude of the artery volume signal at the relevant point corresponds to the maximum value of an amount of change in the artery volume in step ST44. The artery volume signal is obtained as high frequency component of the data output from the artery volume sensor 21, as hereinafter described. The determination on whether corresponding to the maximum value or not in step ST44 is made by whether the amount of change (e.g., derivative value) in the amplitude of the artery volume signal immediately before is a positive value, and the amount of change in the amplitude of the artery volume signal at the relevant point is a negative value.

The CPU 10 proceeds the process to step ST45 when determining as corresponding to the maximum value in step ST44, and proceeds the process to step ST46 when determining as not corresponding to the maximum value.

In step ST45, the CPU 10 stores the maximum value of the artery volume signal (its amplitude) at the relevant point and the pressure value of the air bag of the cuff 2 at the relevant point in the memory 12, and proceeds the process to step ST46. The maximum value of the amplitude of the artery volume signal stored here is handled as the control target value in the feedback control in the electronic manometer 1. If values are already stored in the memory 12, the values are updated to the most recent values.

In step ST46, the CPU 10 determines whether or not the pressure of the air bag of the cuff 2 has reached a predetermined pressure, and proceeds the process to step ST46 when determining as reached and returns the process to step ST42 when determining as not reached.

In step ST47, the CPU 10 confirms the maximum value of the amplitude of the artery volume signal stored in the memory 12 at the relevant point and the pressure value of the air bag of the cuff 2 at the relevant point as the control target value and the control initial cuff pressure, respectively, and returns the process to step ST40 of Fig. 3.

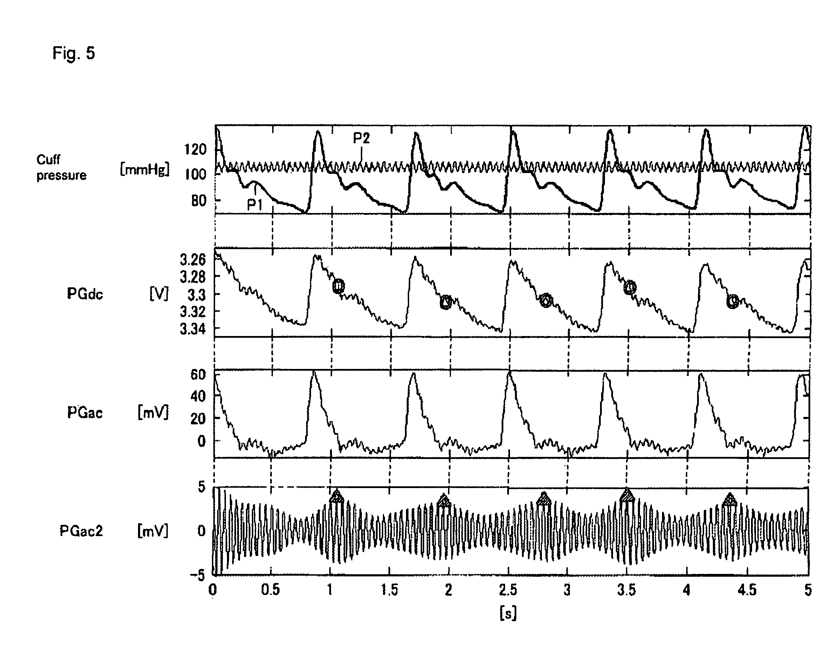

The detection of the control target value in the present embodiment described above will be specifically described with reference to Fig. 5. Fig. 5 is a view showing data handled in the CPU 10 of the electronic manometer 1. Specifically, Fig. 5 shows the pressure to apply on the air bag of the cuff 2 according to the feedback control as P1, the vibration of high frequency superimposed to detect the control target value as P2, the detection output of the artery volume sensor 21 as PGdc, the data removed with DC component of the PGdc as PGac, and the data processed in a band pass filter of the same frequency as the P2 with respect to the PGac as PGac2, and also shows time change of each data.

When the air bag of the cuff 2 is controlled to the pressure at which P2 is superimposed on P1, the amplitude of the PGac2 takes a maximum value (maximal value) at the point the pressure of the air bag of the cuff 2 becomes equilibrium with the intra-arterial pressure. In Fig. 5, triangle mark is given to the maximum value for every heart beat of the PGac2.

In the present embodiment, the control target value stored in the memory 12 is the PGdc (O mark in Fig. 5) at the point the amplitude of the PGac2 takes the maximum value (maximal value). In the present embodiment, the pressure value of the air bag of the cuff 2 at the relevant point is also stored in the memory 12.

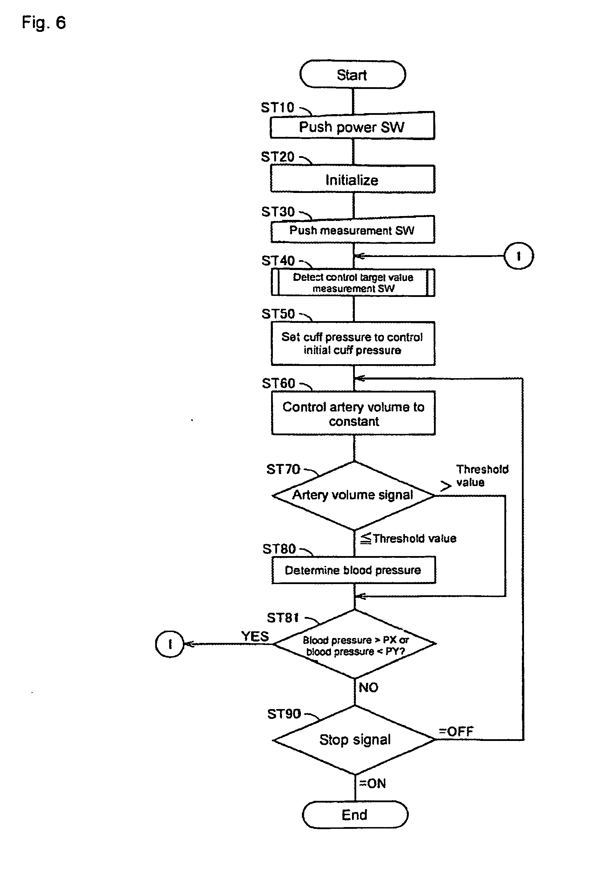

In the embodiment described above, the control target value and the pressure value are detected at the start of measurement of the blood pressure in the electronic manometer 1. The timing of detecting such values in the electronic manometer according to the present invention is not limited to the start of measurement of the blood pressure. For instance, as shown in Fig. 6, whether or not outside a predetermined range (i.e., exceeding a predetermined value PX, or below a specific value PY) is determined for the blood pressure value decided in step ST80 (step ST81), and detection may be made when outside the range (determined as YES in step ST81).

Detection may be made when turning ON the power with respect to the electronic manometer 1, detection may be made when a dedicated switch is arranged on the operation unit 5 and such switch is operated, detection may be made when the pressure of the air bag is suddenly changed during the blood pressure measurement (i.e., when an amount of change in pressure exceeds a certain value defined in advance), or the volume of the artery of the measurement subject detected in the artery volume detection circuit 16 continuously stored in the memory 12 etc., and detection may be made when the value of the artery volume is changed exceeding a constant value defined in advance with respect to the stored value.

In the embodiment described above, the control target value and the pressure value are detected within a period of raising the pressure of the air bag of the cuff 2 (step ST42) in the flowchart shown in Fig. 4. In the example described with reference to Fig. 5, the control target value and the pressure value are detected within a period of lowering the pressure of the air bag of the cuff 2. Thus, in the present invention, the control target value and the pressure value may be detected within the period of raising or within the period of lowering the pressure of the air bag of the cuff 2. However, when detecting within the period of lowering, it at least needs to be a period of lowering after raising the pressure of the air bag of the cuff 2 to greater than or equal to a systolic blood pressure of the measurement subject. To this end, the systolic blood pressure of the measurement subject measured up to now (or input by the measurement subject by operating the operation unit 5) is stored in the memory 12, and the control target value and the pressure value are preferably detected after raising the pressure of the air bag of the cuff 2 to greater than or equal to such blood pressure.

The control target value and the pressure value may be detected with the pressure value of the air bag of the cuff 2 controlled to the pressure value detected before and stored in the memory 12, and with the vibration of high frequency superimposed on the control waveform of the pressure.

As shown in Fig. 4 or Fig. 5, after detecting the control target value and the pressure value with the pressure of the air bag of the cuff 2 raised or lowered while being superimposed with the vibration of high frequency, the control target value and the pressure value are again detected with the pressure of the air bag of the cuff 2 controlled with the detected pressure value and with the pressure controlled so as to superimpose the vibration of high frequency, and the values detected afterward may be stored in the memory 12. The values detected first or afterward may be stored in the memory 12 assuming the detection is successful only when the difference between the values detected first and the values detected afterward is within a predefined range.

As shown in Fig. 5, in the electronic manometer 1, the maximum value of the amplitude of the artery volume signal can be detected for every heart beat of the measurement subject in the artery volume signal. The control target value and the pressure value may be detected for one heart beat, that is, at the point one maximum value of the amplitude of the artery volume signal is obtained, or may be detected by taking an average of each value for a plurality of heart beats.

Each embodiment disclosed herein is illustrative in all aspects, and should not be construed as being restrictive. The scope of the present invention is defined by the claims rather than by the description given above.

### INDUSTRIAL APPLICABILITY

The present invention can be widely used in devices for displaying information related to apnea hypopnea condition, and in particular, is suitable for a device for displaying information to have a user actually feel effects of treatment contributing to an improvement of apnea index at an early stage.

## Claims

1. An electronic manometer (1) comprising:
a cuff (2) attached to a blood pressure measurement site of a measurement subject;
a pressure adjustment unit (14, 15, 31, 32) adapted to adjust a pressure to apply on the cuff;
a pressure detection unit (13, 31) adapted to detect a pressure within the cuff;
an artery volume detection unit (21, 16) adapted to detect a volume of an artery of the measurement subject, which changes by a change in the pressure within the cuff and pulsation; and
a measurement unit (10) adapted to control the volume of the artery of the measurement subject so as to be constant by causing the pressure adjustment unit to increase or decrease the pressure to apply on the cuff based on the change in the volume of the artery of the measurement subject, and measuring a blood pressure of the measurement subject from an increased or decreased value of the pressure to apply on the cuff; wherein
the measurement unit includes,
a pressure maintaining unit (10) adapted to detect the pressure within the cuff at which an amplitude of the change in the volume of the artery of the measurement subject becomes a maximum, and maintaining the pressure,
a feedback control unit (10) adapted to operat the pressure adjustment unit based on the change in the volume of the artery of the measurement subject and feedback controlling so that the amplitude of the change in the volume of the artery of the measurement subject becomes a minimum, and
an extracting unit (10) adapted to extract the pressure within the cuff when the amplitude of the change in the volume of the artery of the measurement subject becomes a minimum by the control from the feedback control unit as the blood pressure of the measurement subject;
the pressure maintaining unit,
causes the artery volume detection unit (21, 16) to detect the volume of the artery of the measurement subject while superimposing a vibration of high frequency during a period of causing the pressure adjustment unit to raise the pressure to apply on the cuff or during a period to lower after raising to greater than or equal to a systolic blood pressure of the measurement subject to detect the pressure within the cuff at which the amplitude of the change in the volume of the artery of the measurement subject becomes a maximum, and
detects the pressure within the cuff (2) at which the amplitude of the change in the volume of the artery of the measurement subject becomes a maximum at a start of measurement of the blood pressure by the measurement unit (10)
**characterized in that** the pressure maintaining unit detects the pressure within the cuff at which the amplitude of the change in the volume of the artery of the measurement subject becomes a maximum at a time other than at the start of measurement during the period of causing the pressure adjustment unit (14, 15, 31, 32) to lower the pressure to apply on the cuff (2) after raising to greater than or equal to the systolic blood pressure of the measurement subject while the feedback controlling is performed by the feedback control unit (10).

2. The electronic manometer according to claim 1, wherein the time other than at the start of the measurement is when the blood pressure measured by the measurement unit (10) exceeds a predetermined value or becomes lower than a specific value.

3. The electronic manometer according to claim 1, wherein the time other than at the start of the measurement is when an amount of change in the volume of the artery of the measurement subject detected by the artery volume detection unit (21, 16) exceeds a constant value during the measurement of the blood pressure by the measurement unit (10).

4. The electronic manometer according to claim 1, wherein the time other than at the start of the measurement is when an amount of change in the pressure within the cuff (2) detected by the pressure detection unit (13, 31) exceeds a certain value during the measurement of the blood pressure by the measurement unit (10).

5. The electronic manometer according to claim 1, further comprising an operation unit (5) adapted to accept an operation from outside; wherein
the time other than at the start of the measurement is when the operation is made to the operation unit (5).

6. The electronic manometer according to claim 1, wherein the pressure maintaining unit (10) detects the pressure within the cuff at which the amplitude of the change in the volume of the artery of the measurement subject becomes a maximum based on a detection result of the volume of the artery of the measurement subject by the artery volume detection unit (21, 16) in a period the pressure adjustment unit adjusts the pressure to apply on the cuff (2) by one heart beat based on the control by the feedback control unit (10).

7. A method of controlling an electronic manometer (1) including a cuff (2) attached to a blood pressure measurement site of a measurement subject, the method causing the electronic manometer (1) to execute the steps of:
detecting a pressure within the cuff (2) at which an amplitude of change in a volume of an artery of the measurement subject becomes a maximum;
maintaining the detected pressure within the cuff (2);
adjusting a pressure to apply on the cuff based on the change in the volume of the artery of the measurement subject while performing a feedback control so that the amplitude of the change in the volume of the artery of the measurement subject becomes a minimum;
extracting the pressure within the cuff (2) when the amplitude of the change in the volume of the artery of the measurement subject becomes a minimum by the feedback control as the blood pressure of the measurement subject; and
wherein
the step of detecting the pressure within the cuff includes detecting the volume of the artery of the measurement subject while superimposing a vibration of high frequency during a period of raising the pressure to apply on the cuff or during a period of lowering after raising the pressure to apply on the cuff to greater than or equal to a systolic blood pressure of the measurement subject to detect the pressure within the cuff at which the amplitude of the change in the volume of the artery of the measurement subject becomes a maximum,
and the step of detecting the pressure within the cuff is executed at a start of blood pressure measurement in the electronic manometer and at a time other than at the start of the measurement during the period of lowering after raising the pressure to apply on the cuff to greater than or equal to the systolic blood pressure of the measurement subject while performing the feedback control.

## Patentansprüche

1. Elektronisches Druckmessgerät (1), umfassend:
eine Manschette (2), die an eine Blutdruckmessstelle eines Messsubjekts angebracht ist;
eine Druckeinstelleinheit (14, 15, 31, 32), die dazu eingerichtet ist, einen Druck, der an die Manschette angelegt werden soll, einzustellen;
eine Druckermittlungseinheit (13, 31), die dazu eingerichtet ist, einen Druck innerhalb der Manschette zu ermitteln;
eine Arterienvolumenermittlungseinheit (21, 16), die dazu eingerichtet ist, ein Volumen einer Arterie des Messsubjekts zu ermitteln, das sich durch eine Änderung des Drucks innerhalb der Manschette und den Pulsschlag ändert; und
eine Messeinheit (10), die dazu eingerichtet ist, das Volumen der Arterie des Messsubjekt derart zu steuern, dass es konstant ist, indem sie die Druckeinstelleinheit dazu veranlasst, den an die Manschette anzulegenden Druck zu erhöhen oder zu erniedrigen, basierend auf der Änderung des Volumens der Arterie des Messsubjekts, und einen Blutdruck des Messsubjekts von einem erhöhten oder verringerten Wert des an die Manschette anzulegenden Drucks zu messen; wobei
die Messeinheit umfasst,
eine Druckaufrechterhaltungseinheit (10), die dazu eingerichtet ist, den Druck innerhalb der Manschette zu ermitteln, an dem eine Amplitude der Änderung des Volumens der Arterie des Messsubjekts ein Maximum annimmt, und den Druck aufrechtzuerhalten,
eine Rückkopplungssteuereinheit (10), die dazu eingerichtet ist, die Druckeinstelleinheit basierend auf der Änderung des Volumens der Arterie des Messsubjekts zu betreiben, und eine Rückkopplungssteuerung derart durchzuführen, dass die Amplitude der Änderung des Volumens der Arterie des Messsubjekts ein Minimum annimmt, und
eine Auswähleinheit (10), die dazu eingerichtet ist, den Druck innerhalb der Manschette als den Blutdruck des Messsubjekts auszuwählen, wenn die Amplitude der Änderung des Volumens der Arterie des Messsubjekts durch die Steuerung der Rückkopplungssteuereinheit ein Minimum annimmt;
die Druckaufrechterhaltungseinheit,
die Arterienvolumenermittlungseinheit (21, 16) dazu veranlasst, das Volumen der Arterie des Messsubjekts zu ermitteln, während sie eine Schwingung von hoher Frequenz während einem Zeitraum, in dem die Druckeinstelleinheit veranlasst wird, den an die Manschette anzulegenden Druck zu erhöhen, oder während einem Zeitraum des Erniedrigens nach Erhöhen auf einen Wert größer oder gleich einem systolischen Blutdruck des Messsubjekts überlagert, um den Druck innerhalb der Manschette zu ermitteln, an dem die Amplitude der Änderung des Volumens der Arterie des Messsubjekts ein Maximum annimmt, und
den Druck zu Beginn der Messung des Blutdrucks durch die Messeinheit (10) innerhalb der Manschette (2) ermittelt, an dem die Amplitude der Änderung des Volumens der Arterie des Messsubjekts ein Maximum annimmt, **dadurch gekennzeichnet, dass** die Druckaufrechterhaltungseinheit den Druck innerhalb der Manschette, an dem die Amplitude der Änderung des Volumens der Arterie des Messsubjekts ein Maximum annimmt, an einem von dem Beginn der Messung unterschiedlichen Zeitpunkt während dem Zeitraum ermittelt, in dem sie die Druckeinstelleinheit (14, 15, 31, 32) veranlasst, den an die Manschette (2) anzulegenden Druck zu erniedrigen, nach Erhöhen auf den Wert größer oder gleich dem systolischen Blutdruck des Messsubjekts, während die Rückkopplungssteuerung durch die Rückkopplungssteuereinheit (10) durchgeführt wird.

2. Elektronisches Druckmessgerät gemäß Anspruch 1, bei dem der von dem Beginn der Messung unterschiedliche Zeitpunkt dann ist, wenn der von der Messeinheit (10) gemessene Blutdruck einen vorgegebenen Wert überschreitet oder niedriger als ein bestimmter Wert wird.

3. Elektronisches Druckmessgerät gemäß Anspruch 1, bei dem der von dem Beginn der Messung unterschiedliche Zeitpunkt dann ist, wenn ein Betrag der Änderung des Volumens der Arterie des Messsubjekts, das von der Arterienvolumenermittlungseinheit (21, 16) ermittelt wird, während der Messung des Blutdrucks durch die Messeinheit (10) einen konstanten Wert überschreitet.

4. Elektronisches Druckmessgerät gemäß Anspruch 1, bei dem der von dem Beginn der Messung unterschiedliche Zeitpunkt dann ist, wenn ein Betrag der Änderung des Drucks innerhalb der Manschette (2), der von der Druckermittlungseinheit (13, 31) ermittelt wird, während der Messung des Blutdrucks durch die Messeinheit (10) einen bestimmten Wert überschreitet.

5. Elektronisches Druckmessgerät gemäß Anspruch 1, ferner umfassend eine Bedienungseinheit (5), die dazu eingerichtet ist, eine Bedienung von außerhalb zu empfangen; wobei
der von dem Beginn der Messung unterschiedliche Zeitpunkt dann ist, wenn die Bedienung an der Bedienungseinheit (5) erfolgt.

6. Elektronisches Druckmessgerät gemäß Anspruch 1, bei dem die Druckaufrechterhaltungseinheit (10) den Druck innerhalb der Manschette, an dem die Amplitude der Änderung des Volumens der Arterie des Messsubjekts ein Maximum annimmt, basierend auf einem Ermittlungsergebnis des Volumens der Arterie des Messsubjekts durch die Arterienvolumenermittlungseinheit (21, 16) in einem Zeitraum, in dem die Druckeinstelleinheit den an die Manschette (2) anzulegenden Druck einstellt, von einem Herzschlag basierend auf der Steuerung der Rückkopplungssteuereinheit (10) ermittelt.

7. Verfahren zur Steuerung eines elektronischen Druckmessgeräts (1), das eine an eine Blutdruckmessstelle eines Messsubjekts angebrachte Manschette (2) umfasst, wobei das Verfahren das elektronische Druckmessgerät (1) dazu veranlasst, die Schritte auszuführen:
Ermitteln eines Drucks innerhalb der Manschette (2), an dem eine Amplitude einer Änderung eines Volumens einer Arterie des Messsubjekts ein Maximum annimmt;
Aufrechterhalten des ermittelten Drucks innerhalb der Manschette (2);
Einstellen eines an die Manschette anzulegenden Drucks basierend auf der Änderung des Volumens der Arterie des Messsubjekts, während eine Rückkopplungssteuerung derart durchgeführt wird, dass die Amplitude der Änderung des Volumens der Arterie des Messsubjekts ein Minimum annimmt;
Auswählen des Drucks innerhalb der Manschette (2) als den Blutdruck des Messsubjekts, wenn die Amplitude der Änderung des Volumens der Arterie des Messsubjekts durch die Rückkopplungssteuerung ein Minimum annimmt; und
wobei
der Schritt des Ermittelns des Drucks innerhalb der Manschette ein Ermitteln des Volumens der Arterie des Messsubjekts während eines Überlagerns einer Schwingung von hoher Frequenz während eines Zeitraums des Erhöhens des an die Manschette anzulegenden Drucks, oder während eines Zeitraums des Erniedrigens, nachdem der an die Manschette anzulegende Druck auf einen Wert größer oder gleich einem systolischen Blutdrucks des Messsubjekts erhöht wurde, umfasst, um den Druck innerhalb der Manschette, an dem die Amplitude der Änderung des Volumens der Arterie des Messsubjekts ein Maximum annimmt, zu ermitteln,
und der Schritt des Ermittelns des Drucks innerhalb der Manschette zu Beginn der Blutdruckmessung in dem elektronischen Druckmessgerät, und zu einem von dem Beginn der Messung unterschiedlichen Zeitpunkt während des Zeitraums des Erniedrigens nach Erhöhen des an die Manschette anzulegenden Drucks auf den Wert größer oder gleich dem systolischen Blutdruck des Messsubjekts ausgeführt wird, während die Rückkopplungssteuerung durchgeführt wird.

## Revendications

1. Manomètre électronique (1) comprenant :
un brassard (2) attaché à un site de mesure de tension artérielle d'un sujet de mesure ;
une unité d'ajustement de pression (14, 15, 31, 32) adaptée pour ajuster une pression à appliquer au brassard ;
une unité de détection de pression (13, 31) adaptée pour détecter une pression dans le brassard ;
une unité de détection de volume d'artère (21, 16) adaptée pour détecter un volume d'une artère du sujet de mesure, qui change par un changement de la pression dans le brassard et de la pulsation ; et
une unité de mesure (10) adaptée pour commander le volume de l'artère du sujet de mesure de manière à ce qu'elle soit constante en amenant l'unité d'ajustement de pression à augmenter ou diminuer la pression à appliquer au brassard sur la base du changement du volume de l'artère du sujet de mesure, et mesurer une tension artérielle du sujet de mesure à partir d'une valeur augmentée ou diminuée de la pression à appliquer au brassard ; dans lequel
l'unité de mesure comprend :
une unité de maintien de pression (10) adaptée pour détecter la pression dans le brassard à laquelle une amplitude du changement du volume de l'artère du sujet de mesure devient maximum, et maintenir la pression,
une unité de commande de rétroaction (10) adaptée pour mettre en oeuvre l'unité d'ajustement de pression sur la base du changement du volume de l'artère du sujet de mesure et effectuer une commande de rétroaction de sorte que l'amplitude du changement du volume de l'artère du sujet de mesure devienne minimum, et
une unité d'extraction (10) conçue pour extraire la pression dans le brassard lorsque l'amplitude du changement du volume de l'artère du sujet de mesure devient minimum par la commande provenant de l'unité de commande de rétroaction en tant que tension artérielle du sujet de mesure ;
l'unité de maintien de pression amène l'unité de détection de volume d'artère (21, 16) à détecter le volume de l'artère du sujet de mesure tout en superposant une vibration haute fréquence pendant une période où l'unité d'ajustement de pression est amenée à augmenter la pression à appliquer au brassard ou pendant une période de diminution après l'augmentation à une pression supérieure ou égale à une tension artérielle systolique du sujet de mesure pour détecter la pression dans le brassard à laquelle l'amplitude du changement du volume de l'artère du sujet de mesure devient maximum, et
détecte la pression dans le brassard (2) à laquelle l'amplitude du changement du volume de l'artère du sujet de mesure devient maximum à un début de mesure de la tension artérielle par l'unité de mesure (10), **caractérisé en ce que** l'unité de maintien de pression détecte la pression dans le brassard à laquelle l'amplitude du changement du volume de l'artère du sujet de mesure devient maximum à un instant autre qu'au début de la mesure pendant la période où l'unité d'ajustement de pression (14, 15, 31, 32) est amenée à diminuer la pression à appliquer au brassard (2) après l'augmentation a une pression supérieure ou égale à la tension artérielle systolique du sujet de mesure alors que la commande de rétroaction est effectuée par l'unité de commande de rétroaction (10).

2. Manomètre électronique selon la revendication 1, dans lequel l'instant autre que le début de la mesure est lorsque la tension artérielle mesurée par l'unité de mesure (10) dépasse une valeur prédéterminée ou devient inférieure à une valeur spécifique.

3. Manomètre électronique selon la revendication 1, dans lequel l'instant autre que le début de la mesure est lorsqu'une quantité de changement du volume de l'artère du sujet de mesure détectée par l'unité de détection de volume d'artère (21, 16) dépasse une valeur constante pendant la mesure de la tension artérielle par l'unité de mesure (10).

4. Manomètre électronique selon la revendication 1, dans lequel l'instant autre que le début de la mesure est lorsqu'une quantité de changement de la pression dans le brassard (2) détectée par l'unité de détection de pression (13, 31) dépasse une certaine valeur pendant la mesure de la tension artérielle par l'unité de mesure (10).

5. Manomètre électronique selon la revendication 1, comprenant en outre une unité d'opération (5) adaptée pour accepter une opération provenant de l'extérieur ; dans lequel
l'instant autre que le début de la mesure est lorsque l'opération est effectuée sur l'unité d'opération (5).

6. Manomètre électronique selon la revendication 1, dans lequel l'unité de maintien de pression (10) détecte la pression dans le brassard à laquelle l'amplitude du changement du volume de l'artère du sujet de mesure devient maximum sur la base d'un résultat de détection du volume de l'artère du sujet de mesure par l'unité de détection de volume d'artère (21, 16) dans une période pendant laquelle l'unité d'ajustement de pression ajuste la pression à appliquer au brassard (2) par un battement cardiaque sur la base de la commande par l'unité de commande de rétroaction (10).

7. Procédé de commande d'un manomètre électronique (1) comprenant un brassard (2) attaché à un site de mesure de tension artérielle d'un sujet de mesure, le procédé amenant le manomètre électronique (1) à exécuter les étapes :
de détection d'une pression dans le brassard (2) à laquelle une amplitude de changement d'un volume d'une artère du sujet de mesure devient maximum ;
de maintien de la pression détectée dans le brassard (2) ;
d'ajustement d'une pression à appliquer au brassard sur la base du changement du volume de l'artère du sujet de mesure tout en effectuant une commande de rétroaction de sorte que l'amplitude du changement du volume de l'artère du sujet de mesure devienne minimum ;
d'extraction de la pression dans le brassard (2) lorsque l'amplitude du changement du volume de l'artère du sujet de mesure devient minimum par la commande de rétroaction en tant que tension artérielle du sujet de mesure ; et
dans lequel
l'étape de détection de la pression dans le brassard comprend la détection du volume de l'artère du sujet de mesure tout en superposant une vibration haute fréquence pendant une période d'augmentation de la pression à appliquer au brassard ou pendant une période de diminution après l'augmentation de la pression à appliquer au brassard à une pression supérieure ou égale à une tension artérielle systolique du sujet de mesure pour détecter la pression dans le brassard à laquelle l'amplitude du changement du volume de l'artère du sujet de mesure devient maximum,
et l'étape de détection de la pression dans le brassard est exécutée à un début de mesure de tension artérielle dans le manomètre électronique et à un instant autre que le début de la mesure pendant la période de diminution après l'augmentation de la pression à appliquer au brassard à une pression supérieure ou égale à la tension artérielle systolique du sujet de mesure tout en effectuant la commande de rétroaction.
